# EUROPÄISCHE PATENTANMELDUNG

(11) **EP 0 972 515 A2**
(43) Veröffentlichungstag der Anmeldung: **19.01.2000**
(21) Anmeldenummer: 99106899.0
(22) Anmeldetag: 08.04.1999
(51) Int. Cl.: A61K 31/165, A61K 31/41, A61K 38/00

(54) **Cyclooxygenase-Inhibitor**

(30) Priorität: 01.07.1998 JP 18623498
(71) Anmelder: A. Nattermann & Cie. GmbH, 50829 Köln (DE)
(72) Erfinder: Tanaka, Zyunji, RD Center Daiichi Seiyaku Co. Ltd., Tokyo 134-8630 (JP)
(74) Vertreter: Lau-Loskill, Philipp, Dipl.-Phys.

(57) **Zusammenfassung**

Es wird ein Cyclooxygenase-2-Inhibitor mit einem Wirkstoff beschrieben, der durch die nachfolgenden Formeln (1) und/oder (1') wiedergegeben ist, 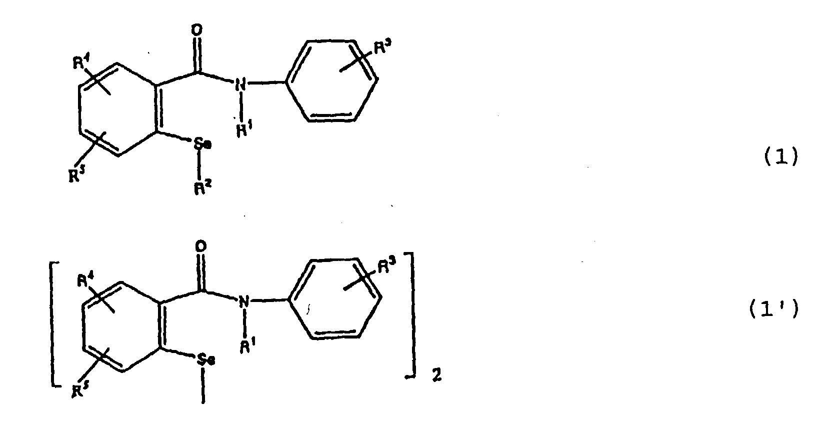 , wobei in den Formeln (1) bzw. (1')
R¹ ein Wasserstoffatom oder eine C₁-C₃-Alkylgruppe;
R² ein Wasserstoffatom, eine Hydroxylgruppe, eine organische Gruppe, die geeignet ist, durch ihre Thioalkoholgruppe innerhalb des Moleküls gebunden zu werden; oder
R¹ und R² miteinander verbunden sind, um eine einzige Bindung auszubilden;
R³ ein Wasserstoffatom, ein Halogenatom, eine C₁-C₃-Alkylgruppe, eine C₁-C₃-Alkoxygruppe, eine Trifluormethylgruppe oder eine Nitrogruppe;
R⁴ und R⁵, die identisch oder unterschiedlich sein können und ein Wasserstoffatom, ein Halogenatom, eine C₁-C₄-Alkoxygruppe, eine Trifluormethylgruppe; oder
R⁴ und R⁵ untereinander unter Ausbildung einer Methylendioxygruppe, einem Salz oder einem Hydrat davon verbunden sind,
bedeuten.

## Beschreibung

Die vorliegende Erfindung betrifft einen Cyclooxygenase-2-Inhibitor, wobei dieser Inhibitor die Synthese von Prostaglandin H und/oder I und/oder die Synthese der Folgemetaboliten Thromboxane verhindert und sowohl als Arzneimittel zur Behandlung als auch zur Verhinderung von ischämischen Erkrankungen, seniler Demenz, Krebs, Asthma, Arteriosklerose und verschiedenen entzündlichen Erkrankungen verwendet werden kann.

Cyclooxygenase (Prostaglandin-Endoperoxide-Synthase) ist ein Enzym, das in vivo die Synthese von Prostaglandin H2 aus ihrem Substrat Arachidonsäure katalysiert. Prostaglandin H2 ist physiologisch sehr aktiv. Aus Prostaglandin H2 werden Metaboliten des Prostaglandins D2, E2 und F2 und die Metaboliten der Thromboxane A2 und B2 hergestellt, wobei diese alle ebenfalls physiologisch sehr aktiv sind.

Folglich führt die Inhibition der Cyclooxygenase-Aktivität zu einer Verhinderung der Synthese von diesen Metaboliten. Von daher können die Prostaglandin H2-Inhibitoren nicht nur Prostaglandin H2 inhibieren sondern auch andere physiologisch sehr aktive Verbindungen, wie zum Beispiel Prostaglandin D2, E2 und F2 und Thromboxane A2 und B2.

Cyclooxygenase ist weiterhin im Zusammenhang mit Entzündungen bekannt. Für die Behandlung von derartigen Entzündungen wurden weithin verschiedene Cyclooxygenase-Inhibitoren verwendet, einschließlich Aspirin und Indomethacin. Jedoch zu dem Zeitpunkt, als diese Arzneimittel entdeckt wurden, war lediglich ein Typ von Cyclooxygenase bekannt, der allgegenwärtig im lebenden Körper anwesend ist.

Ein weiterer Typ von Cyclooxygenase, nämlich der induzierbare Typ von Cyclooxygenase, wurde kürzlich entdeckt. Dieser induzierbare Typ von Cyclooxygenase wird durch viele Stimulanzien hervorgerufen und wird Cyclooxygenase-2 genannt, wobei der allgegenwärtige Typ nachfolgend als Cyclooxygenase-1 bezeichnet wird. Desweiteren wurde kürzlich erkannt, daß Cyclooxygenase-2 grundlegend bei ischämischen Erkrankungen, seniler Demenz, Krebs, Asthma, Arteriosklerose und verschiedenen entzündlichen Erkrankungen involviert ist. Aufgrund dieser Erkenntnisse wird erwogen, daß Cyclooxygenase-2-Inhibitoren potentiell sehr wirksame Arzneimittel zur Behandlung von derartigen Krankheiten darstellen (G. Cirino. Biochem. Pharmacol. 55:105-111, 1998).

Unter Berücksichtigung der vorstehenden Ausführungen liegt der vorliegenden Anmeldung die Aufgabe zugrunde, eine pharmazeutische Zubereitung zur Verfügung zu stellen, die als Wirkstoff eine Verbindung umfaßt, die Cyclooxygenase-2 inhibiert und somit als ein exzellentes therapeutisches Arzneimittel zur Behandlung der vorstehend genannten Erkrankungen dient, die eine geringe Toxizität besitzt und die nur geringe Nebeneffekte so weit hervorruft, daß sie für den klinischen Gebrauch geeignet ist.

Demnach stellt die vorliegende Erfindung einen Cyclooxygenase-2-Inhibitor zur Verfügung, der als Wirkstoff eine Verbindung, die nachfolgend durch die Formeln (1) oder (1') repräsentiert wird, aufweist. 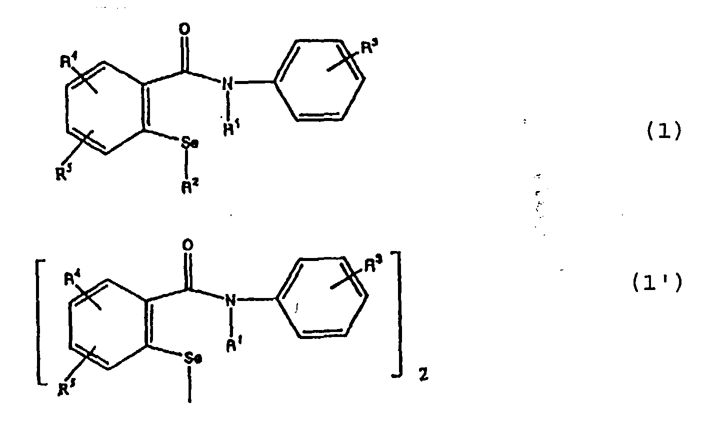 Hierbei bedeuten, daß
R¹ ein Wasserstoffatom oder eine C₁-C₃-Alkylgruppe;
R² ein Peptid oder Protein, das geeignet ist, durch die eigene Thioalkoholgruppe innerhalb des Moleküls gebunden zu werden, oder
R¹ und R² gemeinsam miteinander verbunden sind, um so eine einzige Bindung auszubilden;
R³ ein Wasserstoffatom, ein Halogenatom, eine C₁-C₃-Alkylgruppe, eine C₁-C₃-Alkoxygruppe, eine Trifluormethylgruppe oder eine Nitrogruppe; und
jeweils R⁴ und R⁵, die gleich oder unterschiedlich sein können, ein Wasserstoffatom, ein Halogenatom, eine C₁-C₄-Alkoxygruppe, eine Trifluormethylgruppe oder
R⁴ und R⁵ miteinander verbunden sind und gemeinsam dann eine Methylendioxygruppe, ein Salz oder ein Hydrat hiervon, ausbilden.

Desweiteren betrifft die vorliegende Erfindung einen Cyclooxygenase-2-Inhibitor, der als Wirkstoff 2-Phenyl-1,2-benzisoselenazol-3(2)-one, das nachfolgend auch als Komponente A bezeichnet wird, ein Salz oder ein Hydrat davon, aufweist.

Ferner betrifft die vorliegende Erfindung einen Cyclooxygenase-2-Inhibitor, der als Wirkstoff eine Verbindung umfaßt, die durch die nachfolgende Formel (2) wiedergegeben ist, 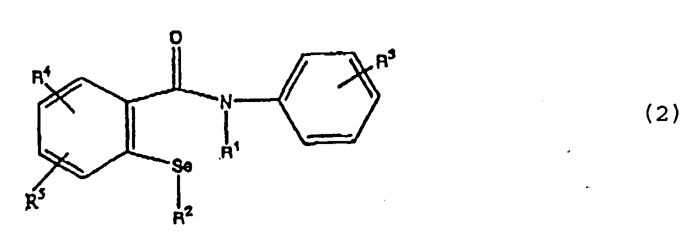 wobei in Formel (2) R² ein Peptid oder ein Protein, das geeignet ist, durch die eigene Thioalkoholgruppe innerhalb des Moleküls angebunden zu werden, bedeuten, und wobei R¹, R³, R⁴ und R⁵ die gleiche Bedeutung besitzen, wie dies vorstehend erläutert ist, einschließlich eines Salzes oder eines Hydrates hiervon.

Darüber hinaus betrifft die vorliegende Erfindung einen Cyclooxygenase-2-Inhibitor, der als Wirkstoff S-(2-Phenylcarbamoyl-phenylselenyl)-Albumin, das nachfolgend auch als Komponente B bezeichnet wird, ein Salz oder ein Hydrat hiervon enthält.

Die vorliegende Erfindung betrifft desweiteren ein Verfahren zur Herstellung einer pharmazeutischen Zubereitung zur Therapie und/oder Prophylaxe von solchen Erkrankungen, deren Ursache in einer Störung und/oder Beeinflussung der Cyclooxygenase-2-Inhibierung liegt, wobei zur Herstellung der Zubereitung als Wirkstoff ein Cyclooxygenase-2-Inhibitor verwendet wird, wie dieser vorstehend beschrieben und durch die allgemeinen Formeln charakterisiert ist.

Desweiteren betrifft die vorliegende Erfindung insbesondere ein Verfahren zur Herstellung einer pharmazeutischen Zubereitung zur Therapie und/oder Prophylaxe von ischämischen Erkrankungen, seniler Demenz, Krebs, Asthma, Arteriosklerose und/oder entzündlichen Erkrankungen, wobei zur Herstellung dieser Zubereitung als Wirkstoff ein Cyclooxygenase-2-Inhibitor der vorstehend genannten Art verwendet wird.

Im Rahmen der vorliegenden Erfindung deckt der Begriff Wirkstoff selbstverständlich nicht nur einen einzelnen Wirkstoff sondern auch eine Wirkstoffmischung ab, die zur Herstellung der erfindungsgemäßen Zubereitung dann entsprechend verwendet wird.

Ferner betrifft die vorliegende Erfindung ein Verfahren zur Therapie und/oder Prophylaxe von solchen Erkrankungen, deren Ursache in einer Störung und/oder Beeinflussung der Cyclooxygenase-2-Inhibierung liegt, wobei eine pharmazeutische Zubereitung, die als Wirkstoff einen Cyclooxygenase-2-Inhibitor der vorstehend beschriebenen Art aufweist, bei einem Erwachsenen in einer täglichen Dosis von 100 bis 2000 mg, bezogen auf den Wirkstoff, oral dargereicht wird.

Insbesondere wird eine solche Zubereitung bei einem Erwachsenen in einer täglichen Dosis oral dargereicht, so daß eine Wirkstoffkonzentration von 200 bis 1000 mg gegeben wird.

Wird das erfindungsgemäße Behandlungsverfahren zur Therapie und/oder Prophylaxe von solchen Erkrankungen, deren Ursache in einer Störung und/oder Beeinflussung der Cyclooxygenase-2-Inhibierung liegt, angewandt, so kann die pharmazeutische Zubereitung, die als Wirkstoff einen Cyclooxygenase-2-Inhibitor der vorstehend genannten Art aufweist, bei einem Erwachsenen in einer täglichen Dosis zwischen 0,05 bis 1000 mg, bezogen auf den Wirkstoff, auch peroral dargereicht wird.

Vorzugsweise wird das erfindungsgemäße Behandlungsverfahren zur Therapie und/oder Prophylaxe von ischämischen Erkrankungen, seniler Demenz, Krebs, Asthma, Arteriosklerose und/oder entzündlichen Erkrankungen angewendet.

Abhängig von der beim erfindungsgemäßen Behandlungsverfahren darzureichenden Wirkstoffkonzentration wird die erfindungsgemäße Zubereitung ein- bis dreimal täglich dargereicht.

Die vorliegende Erfindung wird nachfolgend im Detail beschrieben.

Wie bereits zuvor ausgeführt ist, stellt die vorliegende Erfindung einen Cyclooxygenase-2-Inhibitor zur Verfügung, der, als Wirkstoff, eine Verbindung der Formel (1) enthält. Substituenten für die Verbindung gemäß Formel (1) sind die folgenden:
R¹ ist ein Wasserstoffatom oder eine C₁-C₃-Alkylgruppe, wobei jedoch ein Wasserstoffatom bevorzugt ist.
R² ist ein Peptid oder ein Protein, das geeignet ist, durch seine eigene Thioalkoholgruppe innerhalb des Moleküls gebunden zu werden, oder R¹ und R² sind miteinander verbunden, um so eine einzige Bindung auszubilden.
R³ ist ein Wasserstoffatom, ein Halogenatom, eine C₁-C₃-Alkylgruppe, eine C₁-C₃-Alkoxygruppe, eine Trifluormethylgruppe oder eine Nitrogruppe, wobei hiervon das Wasserstoffatom bevorzugt ist.

Die vorliegende Erfindung stellt desweiteren einen Cyclooxygenase-2-Inhibitor zur Verfügung, der als Wirkstoff eine Verbindung gemäß Formel (2) aufweist. Substituenten für die durch die allgemeine Formel (2) wiedergegebenen Verbindungen sind die folgenden:
R² in der vorstehend genannten Formel (2) bedeutet ein Peptid oder ein Protein, das geeignet ist, durch seine eigene Thioalkoholgruppe innerhalb des Moleküls gebunden zu werden. In bezug auf dieses Protein oder dieses Peptid kann jedes Protein oder Peptid verwendet werden insofern es physiologisch akzeptabel ist, jedoch werden Proteine, die im Serum anwesend sind, wie zum Beispiel insbesondere Albumin und Globulin, bevorzugt. Innerhalb dieser Serumproteine werden insbesondere die Albumine und von den Albuminen insbesondere das menschliche Albumin sehr bevorzugt.

Das Herstellungsverfahren der für die Verbindung A, die in der vorliegenden Erfindung verwendet ist, wurde bereits in der japanischen Patentpublikation (kokoku) No. 2-38591 (zum Beispiel japanische Patentoffenlegungsschrift) (kokai) Nr. 57-67568) offenbart. Das Herstellungsverfahren für die Verbindung B wurde in der offengelegten japanischen Patentanmeldung (kokai) Nr. 7-233056 offenbart.

Entsprechend der bekannten Formulierungstechniken kann die erfindungsgemäße Verbindung A in Form einer Tablette, Kapsel, eines Puders, eines Granulats, eines Sirups oder in Form einer Präparation für die Injektion zusammen mit Additiven, wie beispielsweise einem Trägermittel, einem Bindemittel, einem Zerfalls- bzw. Aufschlußmittel oder einem Lösungsvermittler, aufgemacht sein.

Spezielle Formulierungsbeispiele werden nachfolgend beschrieben.

Im Falle einer Tablette wird insbesondere die nachfolgende Formulierung verwendet:

**Tablette**

| | |
|---|---|
| Verbindung A | 50 mg |
| Carboxymethylcellulose | 25 mg |
| Stärke | 5 mg |
| kristalline Cellulose | 40 mg |
| Magnesiumstearat | 2 mg |
| gesamt | 122 mg |

Die Verbindung A, wenn diese durch typische orale Darreichung oder parenterale Darreichung, so insbesondere als Injektion, angeboten wird, zeigt primäre pharmakologische Effekte. Im Falle der oralen Darreichung liegt die tägliche Dosis der chemischen Verbindung A bei Erwachsenen zwischen 100 und 2.000 mg, vorzugsweise zwischen 200 und 1.000 mg. Dieses Dosis kann abhängig von der Stärke der beim Patienten vorhandenen Symptome angepaßt werden.

Die 2-Phenylcarbamoyl-phenylselenyl-Derivate der vorliegenden Erfindung und deren physiologisch akzeptierbaren Salze werden oral oder parenteral dargereicht. Im Fall der peroralen Darreichung beträgt die Dosis für Erwachsene zwischen 0,05 und 1.000 mg/Tag.

### Toxizität:

Bezüglich der Toxizität der Verbindungen A und B wurden deren LD₅₀-Werte durch Verwendung von Mäusen und Ratten bestimmt. Der LD₅₀-Wert von jeder Verbindung betrug bei einer oralen Darreichung für Mäuse nicht weniger als 6.810 (mg/kg) und lag bei 740 (mg/kg), wenn diese Verbindungen intraperitoneal dargereicht wurde. Im Falle der Ratten waren größere Dosen erforderlich, um einen LD₅₀-Wert zu erreichen. Diese Ergebnisse zeigen, daß das Sicherheitsniveau für die Darreichung derartiger Verbindungen sehr hoch liegt. Eine der Verbindungen, nämlich S-(2-Phenylcarbamoylphenylselenyl)-Albumin, wurde bezüglich der akuten Toxizität getestet. Die Verbindung wurde in physiologischer Kochsalzlösung gelöst und intravenös (5 g/kg) Mäusen dargereicht. Ihr LD₅₀-Wert war größer als 1 g/kg, wodurch das hohe Sicherheitsniveau bestätigt wurde.

### akute Toxizität:

Vier männliche Ratten der Rasse Wistar mit einem Alter von acht Wochen wurden einem akuten Toxizitätstest unterworfen. S-(2-Phenylcarbamoyl-phenylselenyl)-Albumin wurde in physiologischer Kochsalzlösung gelöst und intravenös (1 g/kg/3 ml) dargereicht. Hiernach wurden die Ratten für die nächsten 24 Stunden beobachtet. Innerhalb der Beobachtungsperiode konnte keine besondere Nebenwirkung festgestellt werden und alle Ratten überlebten gesund. Darüber hinaus konnten auch dann keine problematischen Nebenwirkungen beobachtet werden, wenn bei Mäusen als auch Ratten viel höhere Dosen dargereicht wurden.

Unter den zuvor beschriebenen erfindungsgemäßen Verbindungen weist insbesondere S-(2-Phenylcarbamoyl-phenylselenyl)-Albumin die am vielversprechendste Effektivität auf. Prostaglandin H2, synthetisiert mit Cyclooxygenase-2 und seinen Metaboliten, wie z.B. Prostaglandin D2, E2 und F2 und Thromboxan A2 und B2, werden in der Arachidonkastade unterhalb der Arachidonsäure angeordnet. Wie bereits vorstehend erwähnt, sind diese bei ischämischen Erkrankungen, seniler Demenz, Krebs, Asthma, Arteriosklerose und verschiedenen entzündlichen Erkrankungen involviert. Diese Krankheiten können mit S-(2-Phenylcarbamoyl-phenylselenyl)-Derivate selbst sowie mit den physiologisch akzeptablen Salze hiervon behandelt werden, wobei bei ihrer Darreichung exzellente Ergebnisse erzielt werden. Die S-(2-Phenylcarbamoyl-phenylselenyl)-Derivate der vorliegenden Erfindung umfassen sowohl die Verbindung A als auch die Verbindung B.

Durch die an sich bekannten Formulierungstechniken können die erfindungsgemäßen S-(2-Phenylcarbamoyl-phenylselenyl)-Derivate und deren physiologisch akzeptierbare Salze als Tablette, Kapsel, Puder, Granulat, Sirup oder als Präparation für die Injektion, zusammen mit Additiven, wie insbesondere einem Trägermittel, einem Bindemittel, einem Zerfalls- bzw. Aufschlußmittel oder einem Lösungsvermittler, dargereicht werden.

Die 2-Phenyl-1,2-benzisoselenazol-3(2H)-one-Derivate und die 2-Phenylcarbamoyl-Phenylseleny-Derivate, die alle unter die vorliegende Erfindung fallen, wurden in vitro in bezug auf ihre Inhibierungseffekte bei Cyclooxygenase-2-Aktivitäten, die die Synthese von Prostaglandin H2 aus Arachidonsäure katalysieren, getestet. Die Ergebnisse zeigen deutlich erkennbare starke Inhibierungseffekte, die wesentlich stärker sind als von Indomethacin (vergleiche Tabelle 1).

Von daher sind die 2-Phenyl-1,2-Benzisoselenazol-3(2H)-one-Derivate und die 2-Phenylcarbamoyl-Phenylselenyl-Derivate, die alle in den Rahmen der vorliegenden Erfindung fallen, die vorzugsweise als Wirkstoff für Arzneimittel verwendeten Substanzen für die Behandlung der zuvor aufgeführten Erkrankungen, die insbesondere die Synthese von Prostaglandin H2 mit Cyclooxygenase 2 zur Folge haben.

Die vorliegende Erfindung wird nachfolgend im Detail anhand von experimentellen Beispielen erläutert, wobei diese Beispiele nicht den Schutzbereich der vorliegenden Erfindung einschränken sollen.

### experimentelles Beispiel 1

Arachidonsäure wurde als Substrat im folgenden Beispiel verwendet. 10 µl von Arachidonsäure wurde in Methanol (10 mg/ml) gelöst und mit 5 µCi von C¹⁴-markierter Arachidonsäure vermischt. Um ein trockenes Substrat zu erzielen, wurde das Lösungsmittel durch Einblasen von Stickstoff bei Raumtemperatur verdampft. Anschließend wurde das Substrat in 50 µl Dimethylsulfoxid (DMSO) gelöst. Hiernach wurden 10 ml auf 5 mM Tris-HCl-Puffer (pH 8), der 2 mM Phenol enthielt, zu der Substratlösung hinzugeführt, wobei sich hieran eine Schallbehandlung zur vollständigen Lösung der Probe anschloß. Separat hierzu wurden Testverbindungen mit Endkonzentration von 0,1 bis 10 µM hergestellt. In diesem Experiment wurde ein menschliches Serum Albumin-Bindemittel (HAS-binding compound) im vorstehend genannten Puffer gelöst. Die anderen Verbindungen wurden ebenfalls mit DMSO gelöst. 5 µl von jeder Verbindungslösung wurden zu 5 µl der vorstehend beschriebenen Substratlösung hinzugefügt und anschließend vermischt. Die Proben wurden dann präinkubiert bei 35 °C für 10 min. Cyclooxygenase 2, erhalten von Schafplazenten, (Cayman Chem.) wurde getrennt davon mit der 10-fachen Menge des vorstehend genannten Puffers verdünnt, 20 µl der Enzymlösung wurde zu jeder der Proben hinzugefügt. Die Enzymreaktion wurde bei 35 °C während 30 Minuten ausgeführt und durch Hinzufügen von 0,5 ml eiskaltem Ethanol zu jeder Probe gestoppt. Hiernach wurden 2 ml einer 2 %igen Essigsäurelösung zu 0,9 ml einer jeden Probe hinzugefügt. Die Arachidonsäure und ihr Metabolit Prostaglandin H2 wurden dann mit 3 ml Ethylacetat extrahiert. Von jeder extrahierten Probe wurde eine 2,0 ml Teilmenge in ein neues Teströhrchen überführt. Das Lösungsmittel wurde unter reduziertem Druck von den überführten Proben abdestilliert und die Proben wurden im Vakuum getrocknet. Hiernach wurde jede getrocknete Probe in 100 µl Methanol gelöst und 5 µl wurden dann einer hochauflösenden Dünnschichtchromatographie unterworfen.

Arachidonsäure und ihr Metabolit Prostaglandin wurden während der Chromatographie mit einer Entwicklerlösung, bestehend aus Chloroform, Ethylacetat, Methanol, Essigsäure und Wasser im Verhältnis 70:30:8:1:0,5 (V:V), abgetrennt. Die Chromatographieplatte, zusammen mit einer C¹⁴-Standardsubstanz, wurde einer Abbildungsplatte (Fuji-Film) ausgesetzt und hiernach mit einem Fuji-Film Bio-Image Analysierer BAS-2000 analysiert, um so ein Autoradiogramm zu erhalten. Durch Scannen der Flecken in bezug auf die Standardradioaktiven Proben wurde eine Standarddosis-Auswertekurve erzeugt. Hiernach wurde unter Verwendung der Standardkurve jeder eingescannte Wert von Arachidonsäure-Flecken und Prostaglandin H2-Flecken in eine Konzentration von Strahlung transferiert.

Indomethacin wurde von Sigma zur Verfügung gestellt und PZ25 von Rhöne-Poulenc Rorer.

Die Ergebnisse sind in Tabelle 1 wiedergegeben.

**Tabelle 1**

| Cyclooxygenase-Inhibitor 2 | | | | |
|---|---|---|---|---|
| Dosis der Verbindung (µM) | erzeugte Konzentration von Prostaglandin H2 | | | |
| | Verbindung A | Verbindung B | PZ25 | Indomethacin |
| 1 | 91,8 ± 14,3 | 78,33 ± 10,58 | n.b. | n.b. |
| 3 | 78,6 ± 5,28 | 72,13 ± 4,53 ^{*} | 103,25 ± 9,29 | 95,55 ± 7,87 |
| 10 | 46,88 ± 6,49^{**} | 35,40 ± 1,49^{**} | 103,53 ± 11,2 | 99,45 ± 7,80 |
| 30 | 20,10 ± 1,95^{**} | n.b. | 93,9 ± 9,06 | 96,55 ± 0,63 |
| n.b. = nicht bestimmt Signifikanz Niveau der p-Wert: ^{*} P<0,05; ^{**} P<0,01 (kalkuliert nach dem Williams-WilcoxonTest) Verbindung A: 2-Phenyl-1,2-Benzisoselenazol-3(2H)-one Verbindung B: S-(2-Phenylcarbamoyl-Phenylselenyl-Albumin PZ25: 2-Phenyl-1,2-Benzisothiazol-3(2H)-one | | | | |

## Patentansprüche

1. Cyclooxygenase-2-Inhibitor mit einem Wirkstoff, der durch die nachfolgenden Formeln (1) und/oder (1') wiedergegeben ist, 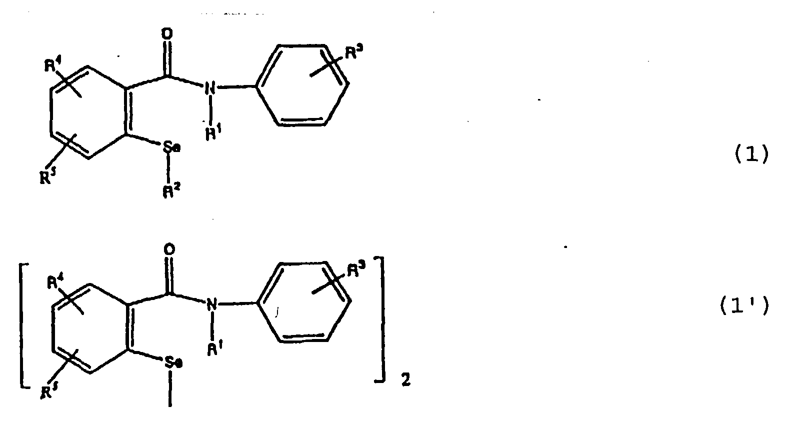 , wobei in den Formeln (1) bzw. (1')
R¹ ein Wasserstoffatom oder eine C₁-C₃-Alkylgruppe;
R² ein Wasserstoffatom, eine Hydroxylgruppe, eine organische Gruppe, die geeignet ist, durch ihre Thioalkoholgruppe innerhalb des Moleküls gebunden zu werden; oder
R¹ und R² miteinander verbunden sind, um eine einzige Bindung auszubilden;
R³ ein Wasserstoffatom, ein Halogenatom, eine C₁-C₃-Alkylgruppe, eine C₁-C₃-Alkoxygruppe, eine Trifluormethylgruppe oder eine Nitrogruppe;
R⁴ und R⁵, die identisch oder unterschiedlich sein können und ein Wasserstoffatom, ein Halogenatom, eine C₁-C₄-Alkoxygruppe, eine Trifluormethylgruppe; oder
R⁴ und R⁵ untereinander unter Ausbildung einer Methylendioxygruppe, einem Salz oder einem Hydrat davon verbunden sind,
bedeuten.

2. Cyclooxygenase-2-Inhibitor nach Anspruch 1, dadurch gekennzeichnet, daß R² ein Peptide, ein Protein oder ein Glykoprotein bedeuten und daß R² geeignet ist, durch seine Thioalkoholgruppe innerhalb des Moleküls gebunden zu werden.

3. Cyclooxygenase-2-Inhibitor nach Anspruch 1 oder 2, dadurch gekennzeichnet, daß R² Albumin, eine Glutathiongruppe oder eine α-Aminosäuregruppe bedeutet, wobei R² geeignet ist, über eine Thioalkoholgruppe innerhalb des Moleküls gebunden zu werden.

4. Cyclooxygenase-2-Inhibitor nach Anspruch 3, dadurch gekennzeichnet, daß das Albumin ein menschliches Albumin ist.

5. Cyclooxygenase-2-Inhibitor nach einem der vorangehenden Ansprüche, dadurch gekennzeichnet, daß es als Wirkstoff 2-Phenyl-1,2-Benzioselenazol-3(2H)-one, ein Salz oder ein Hydrat davon enthält.

6. Cyclooxygenase-2-Inhibitor nach einem der vorangehenden Ansprüche, dadurch gekennzeichnet, daß es S-(2-Phenylcarbamoyl-phenylselenyl)-Albumin, ein Salz oder ein Hydrat davon als Wirkstoff enthält.

7. Verfahren zur Herstellung einer pharmazeutischen Zubereitung zur Therapie und/oder Prophylaxe von solchen Erkrankungen, deren Ursache in einer Störung und/oder Beeinflussung der Cyclooxygenase-2-Inhibierung liegt, wobei zur Herstellung der Zubereitung als Wirkstoff ein Cyclooxygenase-2-Inhibitor nach einem der vorangehenden Ansprüche 1 bis 6 verwendet wird.

8. Verfahren zur Herstellung einer pharmazeutischen Zubereitung zur Therapie und/oder Prophylaxe von ischämischen Erkrankungen, seniler Demenz, Krebs, Asthma, Arteriosklerose und/oder entzündlichen Erkrankungen, wobei zur Herstellung der Zubereitung als Wirkstoff ein Cyclooxygenase-2-Inhibitor nach einem der vorangehenden Ansprüche 1 bis 6 verwendet wird.

9. Verfahren nach Anspruch 7 oder 8, dadurch gekennzeichnet, daß zur Herstellung der Zubereitung als Wirkstoff eine Mischung von Cyclooxygenase-2-Inhibitoren nach einem der Ansprüche 1 bis 6 verwendet wird.

10. Verfahren nach Anspruch 7 bis 9, dadurch gekennzeichnet, daß zur Herstellung der Zubereitung neben dem Wirkstoff oder dem Wirkstoffgemisch noch Trägermittel, Bindemittel, Zerfalls- bzw. Aufschlußmittel und/oder Lösungsvermittler verwendet werden.

11. Verfahren nach Anspruch 7 bis 10, dadurch gekennzeichnet, daß die Zubereitung als Tablette, Kapsel, Puder, Granulat, Sirup oder in Form einer flüssigen Präparation für die Injektion aufgemacht wird.

12. Verfahren zur Therapie und/oder Prophylaxe von solchen Erkrankungen, deren Ursache in einer Störung und/oder Beeinflussung der Cyclooxygenase-2-Inhibierung liegt, dadurch gekennzeichnet, daß eine pharmazeutische Zubereitung, die als Wirkstoff einen Cyclooxygenase-2-Inhibitor nach einem der vorangehenden Ansprüche 1 bis 6 aufweist, bei einem Erwachsenen in einer täglichen Dosis von 100 bis 2000 mg, bezogen auf den Wirkstoff, oral dargereicht wird.

13. Verfahren nach Anspruch 12, dadurch gekennzeichnet, daß die Zubereitung bei einem Erwachsenen in einer täglichen Dosis von 200 bis 1000 mg, bezogen auf den Wirkstoff, oral dargereicht wird.

14. Verfahren zur Therapie und/oder Prophylaxe von solchen Erkrankungen, deren Ursache in einer Störung und/oder Beeinflussung der Cyclooxygenase-2-Inhibierung liegt, dadurch gekennzeichnet, daß eine pharmazeutische Zubereitung, die als Wirkstoff einen Cyclooxygenase-2-Inhibitor nach einem der vorangehenden Ansprüche 1 bis 6 aufweist, bei einem Erwachsenen in einer täglichen Dosis zwischen 0,05 bis 1000 mg, bezogen auf den Wirkstoff, peroral dargereicht wird.

15. Verfahren zur Therapie und/oder Prophylaxe von ischämischen Erkrankungen, seniler Demenz, Krebs, Asthma, Arteriosklerose und/oder entzündlichen Erkrankungen, dadurch gekennzeichnet, daß eine pharmazeutische Zubereitung, die als Wirkstoff einen Cyclooxygenase-2-Inhibitor nach einem der vorangehenden Ansprüche 1 bis 6 aufweist, bei einem Erwachsenen in einer täglichen Dosis von 100 bis 2000 mg, bezogen auf den Wirkstoff, oral dargereicht wird.

16. Verfahren nach Anspruch 15, dadurch gekennzeichnet, daß die Zubereitung bei einem Erwachsenen in einer täglichen Dosis von 200 bis 1000 mg, bezogen auf den Wirkstoff, oral dargereicht wird.

17. Verfahren zur Therapie und/oder Prophylaxe von ischämischen Erkrankungen, seniler Demenz, Krebs, Asthma, Arteriosklerose und/oder entzündlichen Erkrankungen, dadurch gekennzeichnet, daß eine pharmazeutische Zubereitung, die als Wirkstoff einen Cyclooxygenase-2-Inhibitor nach einem der vorangehenden Ansprüche 1 bis 6 aufweist, bei einem Erwachsenen in einer täglichen Dosis zwischen 0,05 bis 1000 mg, bezogen auf den Wirkstoff, peroral dargereicht wird.

18. Verfahren nach einem der Ansprüche 12 bis 17, dadurch gekennzeichnet, daß die Zubereitung in Form von Tabletten, Kapseln, Pudern, Granulaten, Sirups oder in Form einer flüssigen Präparation für die Injektion dargereicht wird.

19. Verfahren nach Anspruch 18, dadurch gekennzeichnet, daß die Zubereitung ein- bis dreimal täglich dargereicht wird.
